# EUROPEAN PATENT APPLICATION

(11) **EP 1 046 338 A1**
(43) Date of publication of application: **25.10.2000**
(21) Application number: 00201779.6
(22) Date of filing: 10.08.1995
(51) Int. Cl.: A01N 63/00, C12R 1/15, C12R 1/01

(54) **Nematicide agent and method for the bio-control of nematodes**

(30) Priority: 10.08.1994 CU 9794
(62) Divisional of application: 95927094.3
(71) Applicant: CENTRO DE INGENIERIA GENETICA Y BIOTECNOLOGIA, Havana (CU); Kambeel, Robert Wilhelmus, 2624 PE Delft (NL)
(72) Inventor: Mena Campos, Jesus, Playa, Havana (CU); Fernandez Machuca, Marina, Playa, Havana (CU); De la Riva de la Riva, Gustavo Alberto, Playa, Havana (CU); Coego Gonzalez, Alberto, Playa, Havana (CU); Vasquez Mendez, Ramon P., Playa, Havana (CU); Garcia Gonzalez, Melba, Playa, Havana (CU); Pimentel Vasquez, Eulogio, Playa, Havana (CU); Lopez Quesada, Alina, Playa, Havana (CU); Garcia Gonzalez, Rolando, Playa, Havana (CU); Zaldua Guerra, Zurima, Playa, Havana (CU); Mencho Ponce, Juan Diego, Playa, Havana (CU)
(74) Representative: Prins, Adrianus Willem

(57) **Abstract**

New bacterial strains, in particular *Sphingobacterium spiritivorum* C-926 and *Corynebacterium paurometabolum* C-924 with useful characteristics in the farming sector due to their nematicidic effect, which can be used in this context for the bio-control of nematodes. Bionematicides capable of effectively controlling phytopathogenic nematode populations in various crops of interest. Useful strains may be isolated from soil taken from areas habitually affected by a high incidence of nematodes, from banana plants showing less severe symptoms of infestation. Strains are selected which are capable of affecting the eggs of the nematode *Meloidogyne incognita,* in experiments carried out under laboratory conditions. Active strains, such as *Sphingobacterium spiritivorum* strain C-926 and *Corynebacterium paurometabolum* strain C-924, are tested to assess the protection which the bacterial preparation, applied directly, can provide to pumpkin and banana plants in relation to *Meloidogyne incognita* and *Radopholus similis.* Strains may also be tested against the zoonematodes *Haemonchus* sp. and *Trichostrongylus* sp. Control of these parasites allows veterinary use. In addition to the nematodes referred to above, and selected due to the high losses which they incur in crops, the strains may also be effective in the control of populations of *Heterodera* ssp., *Globodera* ssp., and *Pratylenchus* ssp., as well as other species of zoonematodes.

## Description

This invention is related to the field of microbiology and in particular to the isolation of two bacterial strains, a *Sphingobacterium spiritivorum* strain and a *Corynobacterium paurometabolum* strain, having useful characteristics in the farming sector due to their nematicidic effect, allowing their use for the bio-control of nematodes in crops or agricultural plants of economic interest.

Nematodes constitute one of the most significant pests in agriculture in tropical, subtropical, and temporate zones (Nickle, W.R., 1991, Manual of Agricultural Nematology, N.Y.). The most important group among the phytopathogenic species is *Meloidogyne* ssp., which causes losses estimated at between 11 and 25% of crops in most tropical areas (Sasser, J.N., 1979, in: Root knot nematodes, Ed. F. Lamberti & C.E. Taylor, Ac. Press, London). There are few options for its control, chemical nematicides being the means most frequently used to control nematode populations. Use is also made, in combination, of the introduction of less susceptible crop varieties, as well as crop rotation strategies and the integrated conduct of harvesting. Nevertheless, the control of phytopathogenic nematodes remains unsatisfactory (Atkinson, H.J., 1993, BCPC Monograph No. 65; Opportunities for Plant Molecular Biology in Crop Production, pages 257-266). The use of chemical nematicidos is limited, since these are systemic substances with a wide spectrum of action as organophosphorics and carbamates, with a high level of toxicity. Another form of attack used against these parasites consists of disinfecting the field, before sowing, with products which are no less toxic, such as methyl bromide and dichloro-propene. All this has given rise to intense studies of the different natural antagonists of nematodes, with a view to their use as bio-preparations which will allow the pest to be controlled while at the same time reducing the risks derived from the excessive use of agricultural chemicals with high toxicity levels.

The nematophageous fungi which inhabit the same environment as the phytonematodes may maintain a predetermined biological balance, and limit to some extent the proliferation of the nematodes. Among other natural predators, we find *Anthrobotrys irregularis;* others are ovicides, such as *Paecilomyces lilacinus* and *Verticillium chlamydosporum.* Studies have also been made of the action of the bacteria *Pasteuria penetrans*, which forms endospores which adhere to the side of the nematode, and, on germination, invade it and cause all the organs of the white nematode to atrophy.

Despite this, the control of nematode populations by these means has continued to be ineffective. Among the reasons which have determined the low rate of effectiveness of the existing methods of biological control are the following:
- Low level of antagonism between the nematode and its antagonist;
- Elevated specificity of the antagonist for a particular kind of nematode;
- Difficulties in obtaining large volumes of the antagonist;
- Difficulties in application due to the nature of the life of the nematode;
- Diversity in the environmental distribution of the nematodes;
- Elevated proliferation of the nematodes.

An object of this invention has been the isolation and characterisation of bacterial strains in the soil which can be used as possible bio-nematicides of a broad spectrum, the use of which will control nematode populations in main agricultural crops. Another object of this invention has been to provide strains which are effective in the control of zoo-nematodes such as *Haemonchus* sp. and *Trichostrongylus* sp., which are indicators of possible use in the veterinary sector.

It was with these objects that new strains were obtained with nematicidal properties. These new strains were classified respectively as *Sphingobacterium spiritivorum* strain C-926, and *Corynobacterium paurometabolum* strain C-924, using the reference systems API 20 NE™ (Ref. 20 090, bioMerieux 69280 Marcy-l'Etoile, France), and API-50 CH (BIOMERIEUX SA, 69280 Marcy-l'Etoile, France).

The above strains were deposited on August 8, 1995, pursuant to the Budapest Treaty with the Centraalbureau voor Schimmelcultures, Baarn, The Netherlands, and received deposit numbers CBS 612.95 (*Sphingobacterium spiritivorum* strain C-926) and CBS 613.95 *(Corynobacterium paurometabolum* strain C-924).

The invention therefore provides new bacterial strains, in particular the *Sphingobacterium spiritivorum* strain C-926 and the *Corynobacterium paurometabolum* strain C-924, which both have useful characteristics in the farming sector due to their nematicidic effect and can be used in this context for the bio-control of nematodes. The invention thus provides bionematicides capable of effectively controlling phytopathogenic nematode populations in various crops of interest.

The invention is not limited to the above two strains which were actually isolated, but also covers mutants thereof and derived strains having essentially similar nematicidic properties, and generally any bacterial strain which can be isolated and selected by a method similar to the method actually used for isolating strains C-926 and C-924.

Useful strains may be isolated from soil taken from areas habitually affected by a high incidence of nematodes, from plants (e.g. banana plants) showing less severe symptoms of infestation. Strains may then be selected which are capable of affecting nematode eggs, e.g. eggs of the nematode Meloidogyne *incognita,* in experiments carried out under laboratory conditions. Active strains, like *Sphingobacterium spiritivorum* strain C-926 and *Corynobacterium paurometabolum* strain C-924, may be tested to assess the protection which they, applied directly, can provide to, e.g. pumpkin and banana plants in relation to, e.g. *Meloidogyne incognita* and *Radopholus similis.* Strains may also be tested against zoonematodes, e.g. Haemonchus sp. and *Trichostrongylus* sp. Control of these parasites allows veterinary use. In addition to the nematodes referred to above, selected because of the high losses which they incur in crops, populations of *Heterodera* ssp., *Globodera* ssp., and Pratylenchus ssp., as well as other species of zoonematodes, may also be controlled effectively by the selected strains.

According to another embodiment of the invention, a nematicidically active metabolite of a selected strain is used rather than a culture of the strain. Said nematicidically active metabolite may be obtained by any useful method, for example be obtained by isolation from its natural environment, or be produced by recombinant DNA technology, or be synthesized by chemical synthesis.

The following examples merely serve to illustrate the invention and should particularly not be construed such as to limit the invention.

### EXAMPLES

### Example 1: Isolation of soil bacterial strains with possible effects on phytopathogenic nematodes.

In an area with a high incidence of nematodes *(Radopholus similis),* banana plants were selected which featured a high infestation in the root system, and others which, despite being in the same area, featured a much lesser infestation of the root system. The difference in the vigour of the two groups was significant. Segments of the roots of the selected plants were collected, of 1 cm in width, as well as samples of the associated soil. The soil samples were screened separately using a No. 40 mesh, and one gram of the screened material was diluted in 10 ml of sterile water, then homogenized by stirring. After being allowed to rest for five minutes, 200 µl of the supernatant was placed on Petri dishes with LB medium. The samples were then incubated for 48 hours at 28°C, and the colonies isolated were then reseeded, but separately, and cultured at 28°C for 24 hours. Comparison of the morphology of the strains derived from each of the groups of samples analyzed resulted in the observation that some of the strains were present only in the group deriving from the soil contained in the root complexes of the more vigorous banana plants, with a healthier root system. In total, four strains were isolated, which were immediately subjected to an analysis of activity with regard to the eggs of *Meloidogyne incognita.*

### Example 2: Classification of the bacterial strain C-926 with nematicidal activity.

The bacterial strain isolated appeared to be a Gram negative coccobacillus, which was classified by the API-20 NE system (BIOMERIEUX SA, 69280 Marcy l'Etoile, France), and designated *Sphingobacterium spiritivorum* C-926 according to that system. The results obtained on the basis of the biochemical tests carried out are shown in Table 1.

**TABLE 1**

| Biochemical test | 24 h | 48 h |
|---|---|---|
| Nitrate (NO₃) reduction | neg | neg |
| Tryptophan (TRP) | neg | neg |
| Glucose fermentation (GLU) | neg | neg |
| Arginine hydrolase (ADH) | neg | neg |
| Urease (URE) | neg | neg |
| Esculine (ESC) | pos | pos |
| Gelatine (GEL) | neg | neg |
| p-Nitrophenyl-βD-Galactopyranoside (PNPG) | pos | pos |
| Assimilation of glucose (GLU) | neg | pos |
| Assimilation of arabinose (ARA) | neg | neg |
| Assimilation of mannose (MNE) | neg | pos |
| Assimilation of mannitol (MAN) | neg | neg |
| Assimilation of N-Acetyl-Glucosamine (NAG) | neg | pos |
| Assimilation of maltose (MAL) | neg | pos |
| Assimilation of gluconate (GNT) | neg | neg |
| Assimilation of caprate (CAP) | neg | neg |
| Assimilation of adipate (ADI) | neg | neg |
| Assimilation of malate (MLT) | neg | neg |
| Assimilation of citrate (CIT) | neg | neg |
| Assimilation of phenyl acetate (PAC) | neg | neg |
| Oxidase activity (OX) | pos | pos |

| Other reactions: | | |
|---|---|---|
| Biochemical test | Notes | |
| Fermentation with production of H₂S | pos | |
| Kliger test | neg | |
| Casein hydrolysis | neg | |
| Catalase activity | pos | |
| Voger-Proskaver test (methyl-carbinol) | neg | |
| Starch hydrolysis | pos | |

Identity number in accordance with the API 20 NE system: 0462304. The identity corresponds to *Sphingobacterium spiritivorum,* the strain being designated as: *Sphingobacterium spiritivorum* C-926.

### Example 3: Classification of the bacterial strain C-924 with nematicidal activity.

The bacterial strain takes the form of a short Gram-positive bacillus, and was classified by the API-50 CH system (BIOMERIEUX SA, 69280 Marcy l'Etoile/France) and was accordingly designated as *Corynobacterium paurometabolum* C-924. The results obtained on the basis of the biochemical tests carried out are shown in Table 2.

**TABLE 2**

| Biochemical test | 24 h | 48 h |
|---|---|---|
| Glycerol | neg | neg |
| Erythritol | neg | neg |
| D-arabinose | neg | neg |
| L-arabinose | neg | neg |
| Ribose | neg | neg |
| D-xylose | neg | neg |
| L-xylose | neg | neg |
| Adonitol | neg | neg |
| β-methyl-xyloside | neg | neg |
| Galactose | neg | neg |
| D-glucose | neg | neg |
| D-fructose | neg | neg |
| D-mannose | neg | neg |
| L-sorbose | neg | neg |
| Ramnose | neg | neg |
| Dulcitol | neg | neg |
| Inositol | neg | neg |
| Mannitol | neg | neg |
| Sorbitol | neg | neg |
| α-methyl-D-mannoside | neg | neg |
| α-methyl-D-glucoside | neg | neg |
| N-acetyl-glucosamine | neg | neg |
| Amygdaline | neg | neg |
| Arbutin | neg | neg |
| Esculin | pos* | |
| Salicine | neg | neg |
| Cellobiose | neg | neg |
| Maltose | neg | neg |
| Lactose | neg | neg |
| Melibiose | neg | neg |
| Saccharose | neg | neg |
| Trehalose | neg | neg |
| Inuline | neg | neg |
| Melecitose | neg | neg |
| D-rabinose | neg | neg |
| Starch | neg | neg |
| Glucogene | neg | neg |
| Xylitol | neg | neg |
| Beta-gentibiose | neg | neg |
| D-turanose | neg | neg |
| D-lyxose | neg | neg |
| D-tagatose | neg | neg |
| D-fucose | neg | neg |
| L-fucose | neg | neg |
| D-arabitol | neg | neg |
| L-arabitol | neg | neg |
| Gluconate | neg | neg |
| 2-keto-gluconate | pos** | |
| 5-keto-gluconate | neg | neg |

Reactions were carried out with the API Coryne system (BIOMERIEUX SA, Marcy l'Etoile/France) and designated according to this system as *Corynobacterium paurometabolum* C-924 :

| Biochemical test | 24 h |
|---|---|
| Reduction of nitrates (NIT) | neg |
| Pyrazinamidase (PYZ) | pos |
| Pyrrolidonyl arylamidase (PyrA) | pos |
| Alkaline phosphatase (PAL) | neg |
| β-glucuronidase (β GUR) | neg |
| β-galactosidase (β GAL) | neg |
| α-glucosidase (α GAL) | neg |
| N-acetyl-β-glucosaminidase (β NAG) | neg |
| Esculin (β-glucosidase) (ESC) | neg |
| Urease (URE) | neg |
| Gelatine (hydrolase) (GEL) | neg |
| Glucose (GLU) | neg |
| Ribose (RIB) | neg |
| Xylose (XYL) | neg |
| Mannitol (MAN) | neg |
| Maltose (MAL) | neg |
| Lactose (LAC) | neg |
| Saccharose (SAC) | neg |
| Glucogene (GLY) | neg |
| Catalase (CAT) | pos |

Identity number according to the Api Coryne system: 024000000000000000004. The identity corresponds to *Corynobacterium paurometabolum,* the strain being designated as: *Corynobacterium paurometabolum* C-924.

### Example 4: Effect of the bacterial strains on the eggs and young larvae of Meloidogyne incognita under "in vitro" conditions.

A number of different bacterial strains were assessed with regard to their effect on the eggs and larvae of *Meloidogyne incognita,* under "in vitro" conditions. The strains to be evaluated were grown in an LB medium at 28°C for 24 hours, under continuous agitation at 100 rpm. The cells were centrifuged, washed with sterile distilled water, and the number of viable specimens was determined in each case. Dilutions of 10⁶ cells/ml were prepared in the LB medium. Also included was a negative control consisting solely of the LB medium. Four variants and four replicas were taken. Each replica was held in a 25 ml capacity "Syracuse" flask. Approximately 1000 eggs of the nematode were added to each receptacle (24 receptacles in total), and 20 ml of the dilutions of each strain, in addition to the negative controls. Periodic observations were made at 24 and 48 hours, measuring the number of eggs which had hatched and the mortality rate among the larvae.

The results of the effects of the bacterial strains C-926 and C-924, expressed as a percentage, are shown in Table 3.

**TABLE 3**

| Strain | Eggs hatched | | | Effect among larvae | | |
|---|---|---|---|---|---|---|
| | 24 h | 48 h | V(%) | 24 h | 48 h | V(%) |
| 1 Strain C-926 | 0 | 0 | 0 | 0 | 0 | - |
| 2 Strain C-924 | 2 | 2 | 0.3 | 2M | 2M | - |
| 3 *E.coli* | 12 | 20 | 5 | 12V | 20V | 5 |
| 4 LB+H₂O | 19 | 25 | 5 | 19V | 25V | 5 |
| Note: V is the coefficient of variation. The hatching levels are considered good for this type of "in vitro" experiment. V = alive, M = dead. | | | | | | |

### Example 5: Effect of various bacterial strains on populations of Meloidogyne incognita in pumpkin plants.

An experiment was conducted making use of pumpkin plants planted in pots of 7 cm in diameter, with a depth of 10 cm of soil previously infected with the eggs of *Meloidogyne incognita.* A black carbonated soil was used, which had been screened and sterilized in an autoclave at 1.5 atmospheres for one hour; and a month was allowed to elapse in order for the microflora of the soil to recover. The inoculation was carried out in each of the replicas (pots), adding 500 viable eggs of the nematode in 2 ml of distilled sterile water. The eggs were deposited uniformly at a depth of 4 cm. The application of the bacterial cultures took place 6 hours subsequent to the inoculation, 50 ml of 10⁷ cells per ml being applied in each case. At 72 hours from application, a pre-germinated pumpkin seed was planted in eacn pot. The humidity of the soil was maintained uniformly in the pots for 60 days by watering. Once this period had elapsed, the plants were extracted, and an assessment made of the damage caused to the root system by the attack of the nematode, according to the Zeck scale. A culture of *E. coli* MC1061 was used as a negative control, in the LB medium without any bacteria. Ten replicas were used. The strain designated as C-926 showed a higher degree of effect in the protection of the root system of the indicator plants. The results are shown in Table 4.

**TABLE 4**

| STRAIN | Mean degree of effect | V(%) |
|---|---|---|
| 1 Strain C-926 | 2.1 | 4 |
| 2 Strain C-924 | 2.0 | 3 |
| *3 E.coli* | 6.0 | 5 |
| 4 LB | 6.5 | 5 |
| Note: V = Coefficient of variation. The hatching levels under the conditions of this type of experiment are always greater than those which are encountered in "in vitro" hatching experiments. | | |

### Example 6: Effect of the bacterial strains on population of Radopholus similis in banana plants.

This experiment was carried out under similar conditions to those described above, in pots 10 cm in diameter and 20 cm in depth. 500 individual *Radolphus similis* were deposited, in 3 ml of distilled sterile water, at a depth of 4 cm. At 5 days, 50 ml of 10⁷ cells/ml of each variant was applied. At 24 hours, a banana plant was planted in each pot. The plants used were derived from an "in vitro" crop, this being free of pathogens. At two months from planting, the plants were transferred to the storage facility, and the humidity of the soil was maintained with two uniform waterings a day. The final evaluation of the experiment was made at the end of five months. The experiment was conducted with seven variants and ten replicas, and the results are shown in Table 5.

**TABLE 5**

| (Mean values per treatment) | | | | | |
|---|---|---|---|---|---|
| STRAIN | Weight (g) Leaf | Weight (g) Root | Nematodes per plant | | |
| | | | Live | Dead | Total |
| 1 *S.spiritivorum* Strain C-926 | 34.7 | 34.2 | 7.5 | 42.5 | 50.0 |
| 2 *C.paurometabolum* Strain C-924 | 36.3 | 36.0 | 15.0 | 15.0 | 20.0 |
| 3 *P.lilacinus** | 36.0 | 34.6 | 105.0 | - | 105.0 |
| 4 *Bacillus subtilis* | 38.55 | 40.6 | 1513.0 | - | 1513.0 |
| 5 LB + water | 27.85 | 25.71 | 9275.0 | - | 9275.0 |
| 6 Sincocin™ | 2.5 | 43.8 | 322.5 | - | 322.5 |
| 7 B.t. kurstaki* | 31.6 | 30.8 | 50.0 | - | 50.0 |

| | | | | | |
|---|---|---|---|---|---|
| * 5 replicas. | | | | | |

The populations of live nematodes were subjected to a statistical analysis (ANOVA), which showed significant differences at the level of 1 to 5% of error:

**TABLE 5a**

| TREATMENTS: | | | | | | |
|---|---|---|---|---|---|---|
| | 3 | 6 | 2 | 7 | 1 | 5 |
| 4 | | | + | ++ | ++ | ++ |
| 3 | | | | + | ++ | ++ |
| 6 | | | | | + | ++ |
| 2 | | | | | + | + |
| 7 | | | | | | |
| 1 | | | | | | |

| | | | | | | |
|---|---|---|---|---|---|---|
| + Significant difference, level 5% of error | | | | | | |
| ++ Highly significant difference, level 1% of error | | | | | | |

### Example 7: Control of Radopholus similis under field conditions, using bio-preparations of nematicidic strains.

An experiment was conducted to verify the control of bio-preparations of *Sphingobacterium spiritivorum* strain C-926 and of *Corynobacterium paurometabolum* strain C-924 on *Radopholus similis,* in a plantation of green bananas of the "Macho 3/4" variety, under field conditions. To this end, a plan was prepared of blocks located at random (7 treatments with three replicas), in three rows of plants, divided into parcels of six plants each. The treatments applied were: *Bacillus subtilis* (negative control used because of its possible occurrence in the ecosystem, at a concentration of 10⁷ cells per ml of culture), Nemacur™ (a chemical nematicide produced and marketed by Bayer AG, which was used in a concentration of 12 litres per hectare at 40%)*, Sphingobacterium spiritivorum* strain C-926, *Corynobacterium paurometabolum* strain C-924, LB culture medium, and the fungus *Paecilomyces lilacinus* (a strain currently used in Cuba for controlling nematodes, and which was used in a concentration of 10⁷ spores per ml of suspension, obtained from cultivation in the solid phase, the dosage recommended by the National Institute of Plant Health). The application of the strains C-924 and C-926 consisted of five litres of a dilution of 10⁷ cells (UFC) per millilitre, derived from cultures previously fermented in LB medium at 28°C, for 18 hours. The phytonematological samples gave results showing variations in the nematode population at two and three months of the treatments, the initial population acting as the base (100%). A second treatment was subsequently carried out and evaluated two months later, comparing the results of the sample with those obtained prior to the first treatment. The results allow to observe the effectiveness of the strain under investigation in the bio-control of *Radopholus similis* in banana plants under field conditions (Table 6) .

**TABLE 6**

| Treatments | 3 months | 6 months | 2 months |
|---|---|---|---|
| A. *B.subtilis* | 155.36% | 182.97% | 128.26% |
| B. C-924+C-926 | 8.15% | 398.52% | 15.56% |
| C. C-924 | 0.00% | 545.69% | 14.98% |
| D. Nemacur™ | 0.14% | 264.00% | 12.35% |
| E. C-926 | 1.19% | 295.70% | 14.96% |
| F. LB medium+water | 257.00% | 1017.91% | 900.02% |
| G. *P.lilacinus* | 70.47% | 232.00% | 188.79% |

### Example 8: Control of Haemonchus sp. using bio-preparations or bacterial strains.

### Collection and disinfection of the eggs:

Samples of the nematode *Haemonchus* sp. were collected from the abdomen of a sheep (ram) slaughtered for the purpose. The females of this adult nematode were treated with "Hibitane" at 0.5% for one minute, and collected in NB medium (nutrient broth) at 37°C for 24 hours. From this point on, all handling was carried out under aseptic conditions within a laminar flow.

The total volume of the NB medium containing the eggs and the remaining nematodes (20 ml) was passed through a 60 µm mesh, followed by one of 30 µm. The eggs of the nematodes were retained on the 30 µm mesh, and the mesh, with the eggs, was introduced into a solution of sodium hypochlorite at 0.1% for three minutes, followed immediately by two washes with sterile distilled water.

### Handling and distribution of the eggs:

The disinfected eggs were extracted from the mesh, and carefully put back into suspension with a solution of Mss medium (minimum volume, in which the weight of the salts was replaced by sugars), consisting of one part by volume of the medium to two parts by volume of distilled sterile water. The volume was adjusted to 500 eggs per ml, and in this manner each sample of 100 µl guaranteed a mean value of 50 eggs. Two culture dishes of 96 wells were used, in which the samples of 100 µl provided were deposited, thus providing the uniform distribution of the nematode eggs.

### Nematicide treatments used:

12 treatments were applied, adjusting the 100 µl samples which were mixed with the 100 µl samples containing the eggs, thus providing 200 µl for each well. Each treatment included 12 replicas.

### Negative controls:

- Medium Mss diluted 2x (treatment #9)
- Sterile distilled water (treatment #5)
- *Bacillus subtilis* strain 6633 (treatment #2)
- *Escherichia coli* strain C-600 (treatment #6)

### Positive controls:

- ** *B.thuringiensis* var. kurstaki H-10 (treatment #4)
- ** *B.thuringiensis* var. israeliensis "Bactimor" (treatment #1)
- ** *B.thuringiensis* var. israeliensis "Tecnar" (treatment #10)

### Nematicides submitted to the test:

- *C-924 (treatment #3)
- *C-926 (treatment #8)
- *C-924+C-926 (treatment #11)
- ***B.thuringiensis* 9452 (treatment #7)
- **B.t. 9499 (treatment #12)

*: Cultures (50 ml) of Mss medium with an initial d.o. = 0.05, cultured in a sieve unit at 28°C, 100 rpm for 54 hours and diluted to 10⁸ UFC/ml.

**: Cultures (50 ml) of Mss medium with initial d.o. = 0.05, cultured in a sieve unit at 28°C, 100 rpm for 5 days (formation of spores) and diluted twice.

The culture dishes were sealed with parafilm, and the eggs in each replica were counted before being incubated at rest at 28°C in darkness, in anticipation of the larvae hatching. The hatched larvae were counted at 36 and 84 hours from the start of the experiment, using an inverted microscope.

**TABLE 7**

| Treatment | T(hours) | Eggs | Larvae | % hatched |
|---|---|---|---|---|
| 1 | 36 | 684 | 8 | 1.2 |
| 1 | 84 | 684 | 9 | 1.3 |
| 2 | 6 | 176 | 64 | 36.4 |
| 2 | 84 | 176 | 65 | 36.9 |
| 3 | 36 | 447 | 46 | 10.3 |
| 3 | 84 | 447 | 69 | 15.4 |
| 4 | 36 | 816 | 0 | 0 |
| 4 | 84 | 816 | 0 | 0 |
| 5 | 36 | 794 | 95 | 12.0 |
| 5 | 84 | 794 | 98 | 12.3 |
| 6 | 36 | 640 | 88 | 13.8 |
| 6 | 84 | 640 | 93 | 14.5 |
| 7 | 36 | 426 | 0 | 0 |
| 7 | 84 | 426 | 0 | 0 |
| 8 | 36 | 562 | 0 | 0 |
| 8 | 84 | 562 | 0 | 0 |
| 9 | 36 | 537 | 129 | 24.0 |
| 9 | 84 | 537 | 132 | 24.6 |
| 10 | 36 | 526 | 0 | 0 |
| 10 | 84 | 526 | 62 | 11.8 |
| 11 | 36 | 492 | 0 | 0 |
| 11 | 84 | 492 | 0 | 0 |
| 12 | 36 | 602 | 0 | 0 |
| 12 | 84 | 602 | 12.8 | 0 |

### Statistical analysis of the results:

Bearing in mind that the larvae hatched between 24 and 48 hours, the statistical analysis (ANOVA) was made on the data at 36 hours.

**TABLE 7A**

| Treatment: | 9 | 6 | 5 | 3 | 1 | 10 | 12 | 7 | 4 | 8 | 11 |
|---|---|---|---|---|---|---|---|---|---|---|---|
| 2 | ++ | ++ | ++ | ++ | ++ | ++ | ++ | ++ | ++ | ++ | ++ |
| 9 | | ++ | ++ | ++ | ++ | ++ | ++ | ++ | ++ | ++ | ++ |
| 6 | | | - | + | ++ | ++ | ++ | ++ | ++ | ++ | ++ |
| 5 | | | | - | ++ | ++ | ++ | ++ | ++ | ++ | ++ |
| 3 | | | | | ++ | ++ | ++ | ++ | ++ | ++ | ++ |
| 1 | | | | | | - | - | - | - | - | - |
| 10 | | | | | | | - | - | - | - | - |
| 12 | | | | | | | | - | - | - | - |
| 7 | | | | | | | | | - | - | - |
| 4 | | | | | | | | | | - | - |
| 8 | | | | | | | | | | | - |

| | | | | | | | | | | | |
|---|---|---|---|---|---|---|---|---|---|---|---|
| - Without significant differences * Significant differences, level of error of 5% ** Highly significant differences, level of error of 1%. | | | | | | | | | | | |

### Notes:

The C-926 (treatment #8) showed a marked effect on the eggs, but this did not occur with the C-924 (treatment #3). The latter case may be due to the longer growing time to which the cultures were subjected (54 hours).

The best controls were selected from this experiment (both positive and negative), and the others were discounted, to be used in later experiments. One of the negative controls which presented difficulties was the *E.coli,* due to its continuous growth, which causes an impediment in the observation of the eggs and larvae. The distilled water was not a very good control either, due to the fact that many of the eggs "slowed down" on being collected in the water, after their treatment with hypochlorite. Sodium hypochlorite does not appear to be the ideal disinfectant for this type of disinfection (despite this having been reported), since the hatching rates (in the negative controls) should be above 50%.

### Example 9: Control of Trichostrongylus sp. using biopreparations of the bacterial strains.

### Collection and disinfection of the eggs:

Collections were made of the nematode *Trichostrongylus* sp. from the abdomen of a sheep (ram) slaughtered for this purpose. The females of this adult nematode were treated with "Hibitane" at 0.5% for one minute, and collected in NB medium (nutrient broth) at 37°C for 24 hours. From this point on, all handling was carried out under aseptic conditions within a laminar flow.

The total volume of the NB medium containing the eggs and the remaining nematodes (20 ml) was passed through a 60 µm mesh, followed by one of 30 µm. The eggs of the nematodes were retained on the 30 µm mesh, and the mesh, with the eggs, was introduced into a solution of sodium hypochlorite at 0.1% for three minutes, followed immediately by two washes with LB medium, diluted 10⁻¹ with sterile distilled water.

### Handling and distribution of the eggs:

The disinfected eggs were extracted from the mesh, and carefully put back into suspension with the 10⁻¹ solution of the LB medium. The volume was adjusted to 100 eggs per ml, and in this manner each sample of 100 µl guaranteed a mean value of 10 eggs. One culture dish of 96 wells was used, in which the samples of 100 µl provided were deposited, thus providing the uniform distribution of the nematode eggs.

### Nematicide treatments used:

12 treatments were applied, adjusting the 100 µl samples which were mixed with the 100 µl samples containing the eggs, thus providing 200 µl for each well. Each treatment included 8 replicas.

### Negative controls:

- LB Medium diluted 10⁻¹ (treatment #5)
- *Bacillus subtilis* strain 6633 [10⁹ UFC/min] (treatment #3)
- *Bacillus subtilis* strain 6633 [10⁸ UFC/min] (treatment #8)

### Positive controls:

- ***B.thuringiensis* kurstaki H-10, diluted 2x (treatment #4)
- ***B.thuringiensis* kurstaki H-10, diluted 10⁻¹ (treatment #9)
- ***B.thuringiensis* kurstaki H-10, diluted 10⁻² (treatment #12)

### Nematodicides submitted to the test:

- *** C-924 [10⁹ UFC/ml] (treatment #1)
- *** C-924 [10⁸ UFC/ml] (treatment #6)
- *** C-924 [10⁷ UFC/ml] (treatment #10)
- *C-926 [10⁹ UFC/ml] (treatment #2)
- *C-926 [10⁸ UFC/ml] (treatment #7)
- *C-926 [10⁷ UFC/ml] (treatment #11)

*: Cultures (50 ml) of LB medium with an initial d.o. = 0.05, cultured in a sieve unit at 28°C, 100 rpm for 46 hours.

**: Cultures (50 ml) of LB medium with initial d.o. = 0.05, cultured in a sieve unit at 28°C, 100 rpm until sporulation.

***: Cultures (50 ml) of LB medium with initial d.o. = 0.05, cultured in a sieve unit at 28°C, 100 rpm for 22 hours.

The culture dish was sealed with parafilm, and the eggs in each replica were counted before being incubated at rest at 28°C in darkness, in anticipation of the larvae hatching.

### Results observed:

Hatched larvae were counted at 48, 120, and 192 hours (8 days) from the start of the experiment, using an inverted microscope. Observation of the larvae was continued subsequently (in the first sieve) with regard to their activity.

**TABLE 8**

| Treatments | % of hatchings | | | % of larvae | |
|---|---|---|---|---|---|
| | 48 h | 120 h | 192 h | Actives | Inactives |
| 1 | 0 | 0 | 0 | - | - |
| 2 | 0 | 0 | 0 | - | - |
| 3 | 0 | 15.2 | 15.2 | 100 | 0 |
| 4 | 0 | 0 | 1.8 | 0 | 100 |
| 5 | 48.3 | 48.3 | 48.3 | 100 | 0 |
| 6 | 0 | 0 | 3.12 | 100 | 0 |
| 7 | 0 | 6.2 | 12.5 | 66.6 | 33.3 |
| 8 | 4.6 | 25.5 | 25.5 | 100 | 0 |
| 9 | 0 | 0 | 0 | - | - |
| 10 | 25.7 | 50 | 50 | 14.3 | 85.7 |
| 11 | 1.5 | 1.5 | 1.5 | 0 | 100 |

### Notes:

The strains C-924 (treatments #1, 6 and 10) and C-926 (treatments #2, 7, and 11) showed a marked effect on the eggs in higher concentrations, but this did not occur with the lower concentration, where the inactive response of the larvae could be observed. The joined effect on the eggs and larvae of the first stage should be taken into account. Although sodium hypochlorite does not appear to be the ideal disinfectant for this type of work, the percentages of hatching of the negative controls were acceptable. The growing time of these strains is a very important factor to take into account, as can be appreciated by comparing the results of experiment #1 with those of experiment #2.

### Example 10: Control of Trichostrongylus sp. using biopreparations of the bacterial strains taken at different growing times.

### Collection and disinfection of the eggs:

Collections were made of the nematode *Trichostrongylus* sp. from the abdomen of a sheep (ram) slaughtered for this purpose. The females of this adult nematode were treated with "Hibitane" at 0.5% for one minute, and collected in an LB medium diluted at 10⁻¹ at 37°C for 24 hours. From this point on, all handling was carried out under aseptic conditions within a laminar flow. The total volume of the LB medium containing the eggs and the remaining nematodes (20 ml) was passed through a 60 µm mesh, followed by one of 30 µm. The eggs of the nematodes were retained on the 30 µm mesh, and the mesh, with the eggs, was introduced into a solution of "Hibitane" at 0.5% for three minutes, followed immediately by three washes with LB medium, diluted 10⁻¹.

### Handling and distribution of the eggs:

The disinfected eggs were extracted from the mesh, and carefully put back into suspension with the 10⁻¹ solution of the LB medium.

The volume was adjusted to 300 eggs per ml, and in this manner each sample of 100 µl guaranteed a mean value of 30 eggs.

One culture dish of 96 wells was used, in which the samples of 100 µl provided were deposited, thus providing the uniform distribution of the nematode eggs.

### Nematodicide treatments used:

16 treatments were applied, with 6 replicas, adjusting the 100 µl samples which were mixed with the 100 µl samples containing the eggs, thus providing 200 µl for each well.

### Negative controls used:

- LB medium diluted 10⁻¹
- **B.subtilis* strain 6633 [10⁷ UFC/ml], 8 hours growth
- **B.subtilis* strain 6633 [10⁸ UFC/ml], 16 hours growth
- **B.subtilis* strain 6633 [10⁸ UFC/ml], 20 hours growth
- **B.subtilis* strain 6633 [10⁸ UFC/ml], 24 hours growth
- **B.subtilis* strain 6633 [10⁸ UFC/ml], 36 hours growth

### Nematicide treatments applied to the experiment:

- *C-924 [10⁷ UFC/ml], 8 hours growth
- *C-924 [10⁸ UFC/ml], 16 hours growth
- *C-924 [10⁸ UFC/ml], 20 hours growth
- *C-924 [10⁸ UFC/ml], 24 hours growth
- *C-924 [10⁸ UFC/ml], 36 hours growth
- *C-926 [10⁷ UFC/ml], 8 hours growth
- *C-926 [10⁸ UFC/ml], 16 hours growth
- *C-926 [10⁸ UFC/ml], 20 hours growth
- *C-926 [10⁸ UFC/ml], 24 hours growth
- *C-926 [10⁶ UFC/ml], 36 hours growth

* Cultures (50 ml) of LB medium with initial d.o. = 0.05, cultured in a sieve at 28°C at 100 rpm.

The culture dish was sealed with parafilm, and the eggs in each replica were counted before being incubated at rest at 28°C in darkness, in anticipation of the larvae hatching.

### Results observed:

Counts were made of hatched larvae every 24 hours, up to 168 h (during one week), and observations made to determine the moment at which hatching occurred in each treatment.

**TABLE 9**

| Culture time: | 8h | | | 16h | | | 20h | | | 24h | | | 36h | | |
|---|---|---|---|---|---|---|---|---|---|---|---|---|---|---|---|
| Treatment | H: | L: | % | H: | L: | % | H: | L: | % | H: | L: | % | H: | L: | % |
| *C.paurometabolum* Strain C-924* | 167 | 6 | 3.5% | 161 | 0 | 0% | 154 | 0 | 0% | 167 | 0 | 0% | 266 | 0 | 0% |
| *S. spiritivorum* Strain C-926** | 148 | 69 | 46.6% | 265 | 149 | 56.2% | 172 | 104 | 60.4% | 149 | 49 | 32.8% | 169 | 64 | 37.8% |
| *B.subtilis**** | 112 | 71 | 63.3% | 166 | 107 | 64.4% | 182 | 107 | 58.7% | 167 | 113 | 67,6% | 217 | 160 | 73.7% |
| CONTROL: LB diluted at 10⁻¹ = Eggs: 192. Hatched larvae: 142, making 79.9 % hatching. | | | | | | | | | | | | | | | |

| | | | | | | | | | | | | | | | |
|---|---|---|---|---|---|---|---|---|---|---|---|---|---|---|---|
| * Final count at 168 hours | | | | | | | | | | | | | | | |
| ** Hatching took place as from 110 hours of incubation | | | | | | | | | | | | | | | |
| *** Hatching was prior to 48 hours. | | | | | | | | | | | | | | | |

### Notes:

The strains C-924 and C-926 showed a marked effect on the eggs at different times of growth; however, it is quite clear that C-924 entirely limits hatching as from 16 hours of growth, while the effect of C-926 consists of retarding hatching up to later than 110 hours, basically in the treatments of 24 and 36 hours of growth. This delay can be effective for the control of nematodes "in vivo", by breaking the normal hatching cycle, which is between 24 and 48 hours (confirmed in the negative controls of this experiment).

It was shown by this experiment that the growth time of the strains is a very important factor to take into account, as we have already indicated by comparing the results of experiment #1 with that of #2.

"Hibitane" turns out to be an adequate disinfectant for this type of work, inasmuch as the hatching percentages in the negative controls were between 58.7 and 79.9%.

## Claims

1. A method for the bio-control of nematodes, comprising contacting the soil, a plant or a seed with an effective quantity of a nematicidic agent, the said agent being a culture obtained from *Sphingobacterium spiritivorum* C-926 and/or *Corynobacterium paurometabolum* C-924, or a metabolite derived from the said strains, obtained by natural, recombinant or synthetic route, in an appropriate carrier, or using the said nematicidic agent in a veterinary composition for the control of zoonematodes.

2. A method according to Claim 1, characterised by the fact that it is used for the control of *Radopholus similis* and *Meloidogyne incognita* in plants, and for the zoonematodes *Haemonchus* ssp. and Trichostrongylus ssp.

3. A method according to Claim 1, characterised by the fact that, for the situation in which it is applied on the soil, the effective quantity of the nematicidic agent consists of approximately 14 L of a culture of *Sphingobacterium spiritivorum* C-926 and/or *Corynobacterium paurometabolum C-924,* which is added per hectare of ground in an approximate concentration of between 10⁶ and 10⁷ of colony forming units per ml of medium, which are applied to the affected ground every three months.

4. A method according to Claim 1, characterised by the fact that it is effective in the control of *Radopholus similis* and *Meloidogyne incognita* in plants and the zoonematodes *Haemonchus* ssp. and *Trichostrongylus* ssp.

5. A culture of *Sphingobacterium spiritivorum,* used as a nematodicidic agent in the method according to Claim 1, characterised by the fact that the said culture is of the strain C-926 of *Sphingobacterium spiritivorum* or any mutation derived therefrom which is active against nematodes.

6. A culture of *Corynobacterium paurometabolum,* used as a nematodicidic agent in the method according to Claim 1, characterised by the fact that the said culture is of the strain C-924 of *Corynobacterium paurometabolum* or any mutation derived therefrom which is active against nematodes.

7. A culture according to Claim 4, characterised by the fact that it contains between approximately 10⁶ and 10⁷ colony forming units per ml of the culture medium.

8. A nematicidic agent used for the biological control of nematodes, characterised by the fact that the culture contains at least one strain of *Sphingobacterium spiritivorum* and/or *Corynobacterium paurometabolum,* or a mutation derived from the said strains, as well as any active principle agent or metabolite obtained on the basis of the said strains, obtained by natural, recombinant or synthetic route, and an appropriate carrier, to be used in agriculture or in the veterinary sector.

9. A nematicidic agent according to Claim 7, characterised by the face that, for the control of phytonematodes, the said appropriate carrier is a fertilizer, a pre-packaged soil, a seed cover device, a powder, a granulate, a nebulizer, a suspension, or a liquid, or any of the variants indicated, in an encapsulated form.

10. Method for obtaining a bacterial strain having nematicidal properties, comprising collecting samples of soil and/or plant tissue from an area affected by nematodes, distinguishing samples cf highly affected material from samples of less affected material, isolating bacterial strains present in said samples, comparing bacterial strains present in highly affected samples with bacterial strains present in less affected samples, selecting bacterial'strains present only in said less affected samples, testing nematicidal properties of the selected strains and selecting those bacterial strains having useful nematicidal properties.

11. A bacterial strain having nematicidal activity, which is obtainable by the method of claim 10.

## Amended claims

### Amended claims in accordance with Rule 86(2) EPC.

**1.** A method for the bio-control of nematodes, comprising contacting soil, a plant or a seed with an effective quantity of a nematicidic agent, the said agent being a culture obtained from *Sphingobacterium spiritivorum* strain C-926, or a metabolite derived from the said strain, obtained by natural, recombinant or synthetic route, in an appropriate carrier.

**2.** A method according to Claim 1, wherein the nematicidic agent is used for the effective control of *Radopholus similis* and *Meloidogyne incognita* in plants.

**3.** A method according to Claim 1 or Claim 2, wherein the nematicidic agent comprises a culture of *Sphingobacterium spiritivorum* strain C-926, which is added in a concentration of between 10⁶ and 10⁷ colony forming units (CFU) per ml of medium, which are applied to the affected ground every three months at approximately 14 liters per ha.

**4.** Use of a nematicidic agent for the manufacture of a veterinary composition for the control of zoonematodes, wherein said nematicidic agent is a culture obtained from *Sphingobacterium spiritivorum* strain C-926, or a metabolite derived from the said strain, obtained by natural, recombinant or synthetic route, in an appropriate carrier.

**5.** Use according to Claim 4 wherein the nematicidic agent is used for the manufacture of a veterinary composition for the effective control of the zoonematodes *Haemonchus* ssp. and *Trichostrongylus* ssp. in animals.

**6.** A culture of *Sphingobacterium spiritivorum,* wherein the said culture is of the strain C-926 of *Sphingobacterium spiritivorum* or any mutation derived therefrom which is active against nematodes.

**7.** A culture according to Claim 6, which contains between 10⁶ and 10⁷ colony forming units (CFU) per ml of culture medium.

**8.** A nematicidic agent for effective biological control of nematodes in plants, which comprises a culture containing *Sphingobacterium spiritivorum* strain C-926, or a mutation derived from the said strain, as well as any active principle compound or metabolite obtained on the basis of the said strain by natural, recombinant or synthetic route, and an appropriate carrier.

**9.** A nematicidic agent according to Claim 8 for the control of phytonematodes, wherein the said appropriate carrier is a fertilizer, a pre-packaged soil, a seed cover device, a powder, a granulate, a nebulizer, a suspension, or a liquid, or any of the variants indicated, in an encapsulated form.

**10.** A nematicidic agent for effective biological control of nematodes in animals, which comprises a culture containing *Sphingobacterium spiritivorum* strain C-926, or a mutation derived from the said strain, as well as any active principle compound or metabolite obtained on the basis of the said strain by natural, recombinant or synthetic route, and an appropriate carrier.
